# EUROPÄISCHE PATENTSCHRIFT

(11) **EP 2 373 633 B1**
(45) Veröffentlichungstag und Bekanntmachung des Hinweises auf die Patenterteilung: **16.04.2014**
(21) Anmeldenummer: 09796353.2
(22) Anmeldetag: 07.12.2009
(51) Int. Cl.: C07D 251/70, C09D 7/12, C09D 167/00, C08K 5/205, C08K 5/3492

(54) **VERFAHREN ZUR HERSTELLUNG VON TRIAZINCARBAMATEN UNTER VERWENDUNG VON CHLORFORMIATEN**
METHOD FOR PRODUCING TRAZINE CARBAMATES USING CHLOROFORMATES
PROCÉDÉ DE PRÉPARATION DE CARBAMATES DE TRIAZINE PAR UTILISATION DE CHLOROFORMIATES

(30) Priorität: 09.12.2008 DE 102008061139
(43) Veröffentlichungstag der Anmeldung: 12.10.2011
(73) Patentinhaber: Borealis Agrolinz Melamine GmbH, 4021 Linz (AT)
(72) Erfinder: DICKE, René, A-4060 Leonding (AT); BURGER, Martin, 84032 Altdorf (DE); ENDESFELDER, Andreas, A-4020 Linz (AT); HAHN, Christoph, A-4040 Linz (AT); POSER, Sven, 06712 Breitenbach (DE); FRANK, Willy, 06246 Bad Lauchstädt (DE); ARNOLD, Manfred, 06667 Leißling (DE)
(74) Vertreter: Morawski, Birgit
(86) Internationale Anmeldenummer: PCT/EP2009/066538
(87) Internationale Veröffentlichungsnummer: WO 2010/066683

(56) Entgegenhaltungen:
- EP-A1- 0 559 044
- EP-A2- 0 226 536
- US-A- 6 063 922
- DUYNSTEE E ET AL: "Triazine Isocyanates Part II: Reactions of 2,4-diamino-s-triazines with Ethyl Chlorocarbonate" RECUEIL DES TRAVAUX CHIMIQUES DES PAYS-BAS, ELSEVIER SCIENCE PUBLISHERS, AMSTERDAM, NL, Bd. 81, 1. Januar 1962 (1962-01-01), Seiten 241-254, XP009131105 ISSN: 0165-0513
- KITAJIMA H ET AL: "Reactions of melamine and bezoguanamine with ethyl chlorocarbonate" YUKI GOSEI KAGAKU KYOKAISHI - JOURNAL OF SYNTHETIC ORGANIC CHEMISTRY. JAPAN, YUKI GOSEI KAGAKU KAOKAI, TOKYO, JP, Bd. 32, 1. Januar 1974 (1974-01-01), Seiten 723-726, XP009131041 ISSN: 0037-9980

## Beschreibung

Die vorliegende Erfindung betrifft ein Verfahren zur Herstellung von Triazincarbamten nach Anspruch 1.

Die US5,084,541 beschreibt Tricarbamoyltriazine (Triazintricarbamate, Melamintricarbamate), die ausgehend von Triazintriisocyanat durch Umsetzung mit verschiedenen Alkoholen synthetisiert werden. Diese stets dreifach substituierten Produkte können beispielsweise als Vernetzer eingesetzt werden. Nachteil dieses Verfahren ist, dass das Isocyanat als eine hochreaktive Zwischenstufe isoliert werden muss. Auch können auf diese Weise maximal Triazin-tri-carbamate hergestellt werden.

Die DE102004018543 A1 beschreibt ebenfalls carbamatgruppenhaltige Triazinderivate, die als Vernetzer für Lacke mit verbesserten Eigenschaften dienen. Auf die Herstellung der Carbamate wird nicht weiter eingegangen.

Die DE10259672 beschreibt die Herstellung von Alkoxycarbonylamino-triazinen durch die Umsetzung von Di- und Triamino-trizinen mit cyclischen Carbonaten, wobei aber große Mengen an Base benötigt werden.

Die US 6,063,922 beschreibt Triazincarbamate, die aus einer Umsetzung mit Melamin und acyclischen organischen Carbonaten in Gegenwart von starken Basen gebildet werden. Dabei werden stets Bi- oder Tricarbamate gebildet. Nachteilig ist bei diesem Verfahren die Verwendung einer Base, die in großen Mengen zugegeben werden muss, um ausreichende Umsätze zu erzielen. Auch wird die Umsetzung von Melamin mit Butylchlorformiat in Anwesenheit von alkoholischem Natriumbutoxid beschrieben. Allerdings liefert diese Umsetzung nur geringe Ausbeuten, da es zu zahlreichen Nebenreaktionen kommen kann.

EP 0 559 044 A1 beschreibt die Herstellung von herbiziden Sulfonylharnstoffsalzen mittels einer zweistufigen Reaktion. In der zweiten Reaktionsstufe werden Sulfonamidsalte (IIa) mit Carbamten (III) zu Sulfonylharnstoffsalzen (I) umgesetzt. Die verwendeten Carbamate werden durch Umsatz von Triazinen (IV) mit maximal zwei substituierten Aminoresten mit Chlorameisensäureestern erhalten. Die Reaktion erfolgt in Anwesenheit von Natriumhydrogencarbonat oder Kaliumhydrogencarbonat.

EP 0 226 536 A2 beschreibt die Herstellung von (Di)alkoxycarbonyl- Amino-s-Triazinderivaten und deren Verwendung als Insektizide. Diese werden durch Umsetzung von Triazinderivaten mit maximal zwei substituierten Aminoresten mit einem reaktionsfähigen Säurederivat, wie zum Beispiel einem Säurehalogenid, in Gegenwart einer Base, wie zum Beispiel Alkali- und Erdalkalimetallcarbonate und -hydrogencarbonate erhalten.

Aufgabe der Erfindung war es ein neues Verfahren zu entwickeln, das einfach durchzuführen ist, gute Ausbeuten, bevorzugt über 90%, liefert und die oben erwähnten Nachteile vermeidet.

Es hat sich nun überraschenderweise gezeigt, dass die Herstellung von mindestens einem Triazincarbamat der Formel I 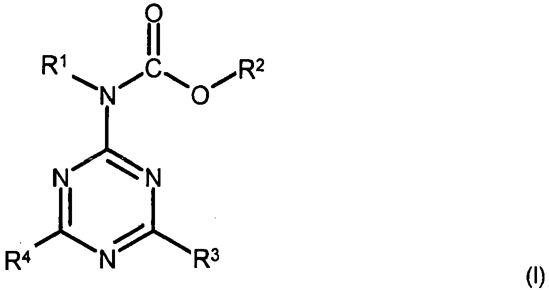 oder deren Mischungen, wobei
**R³** einen mit seinem zentralen Stickstoffatom an ein C-Atom des Triazinringes der Struktur der Formel (I) gebundenen Rest der Formel **R⁵-N-R⁶** bedeutet,
**R⁴** einen mit dem Stickstoffatom an ein C-Atom des Triazinringes der Struktur der Formel (I) gebundenen Rest der Formel **R⁷-N-R⁸** bedeutet,
**R⁶** und **R⁸** unabhängig voneinander H, Q², -CO-**O-R²,** -CO-**R⁹** oder -CO-O-**R¹⁰** und
**R¹, R⁵** und **R⁷** unabhängig voneinander Q², -CO-**O-R²**, -CO-**R⁹** oder -CO-O-**R¹⁰** bedeuten, wobei
- Q² jeweils ein lineares oder verzweigtes C₁-C₅₀-Alkyl, C₅-C₂₀-Cycloalkyl, C₅-C₂₀-Aryl, C₁-C₅₀-alkylsubstituiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₂₀-Alkenyl, C₂-C₁₂-Alkinyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH-und/oder -OC(O)O- unterbrochen sein kann, ist,
**R** ein lineares oder verzweigtes C₁-C₅₀-Alkyl, C₅-C₂₀-Cycloalkyl, C₅-C₂₀-Aryl, C₁-C₅₀-alkylsubstituiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₂₀-Alkenyl, C₂-C₁₂-Alkinyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH- und/oder -OC(O)O- unterbrochen sein kann und/oder ein oder mehrere Halogenatome und/oder Nitrogruppen als Substituenten aufweist, ist,
**R⁹** einen Rest der allgemeinen Formel (II) 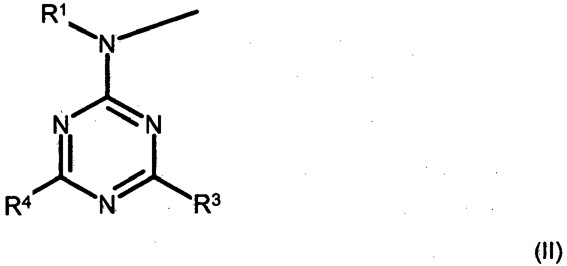 bedeutet,
**R¹⁰** einen Rest der allgemeinen Formel (III) 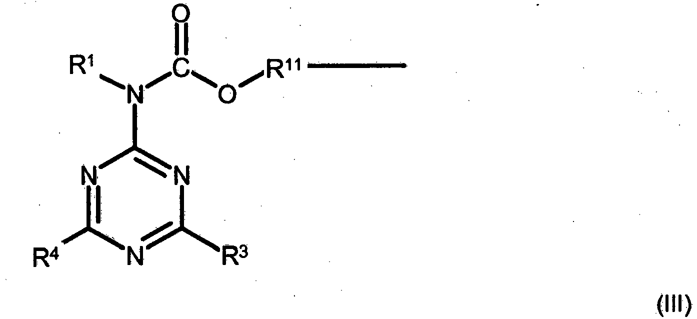 bedeutet, wobei
- **R¹¹** jeweils ein lineares oder verzweigtes C₁-C₅₀-Alkyl, C₅-C₂₀-Cycloalkyl, C₅-C₂₀-Aryl, C₁-C₅₀-alkylsubstituiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₂₀-Alkenyl, C₂-C₁₂-Alkinyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH-und/oder -OC(O)O- unterbrochen sein kann, ist,
durch Umsetzung von mindestens einem Triazin der Formel IV 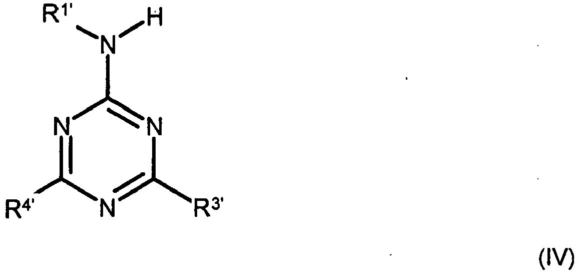 wobei R^{1'} die Bedeutung von R¹, R^{3'} die Bedeutung von R³ und R^{4'} die Bedeutung von R⁴ aufweist
mit mindestens einem Chlorformiat der allgemeinen Formel (V) 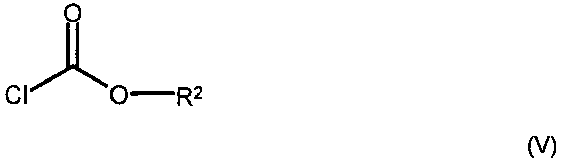 und/oder der allgemeinen Formel (VI) 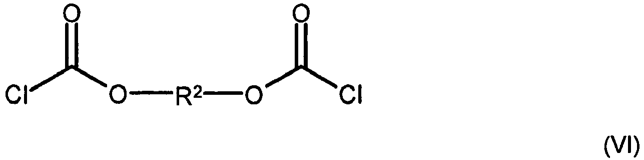 in Gegenwart mindestens einer Alkali- oder Erdalkalimetallverbindung aus der Gruppe umfassend NaHCO₃, KHCO₃, Na₂CO₃, K₂CO₃, MgCO₃, CaCO₃, Na₃PO₄, Na₂HPO₄, Na-acetat, Dinatriumoxalat, Butyllithium, Methyllithium, Phenyllithium, Methylnatrium, Butylnatrium, Phenylnatrium, Methylmagnesiumbromid, LiAlH₄, Natriumamid, möglich ist, wobei die Alkali- oder Erdalkalimetallverbindung nicht in Form eines Alkoholates vorliegt.

Wie hierin verwendet, bezeichnet der Begriff "C₁-C₅₀-Alkyl" Reste, wie Methyl, Ethyl, n-Propyl, Isopropyl, n-Butyl, Isobutyl, s-Butyl, t-Butyl, Amyl, t-Amyl, Hexyl, Heptyl, Octyl, Nonyl, Decyl, Undecyl, Dodecyl, und längerkettige Reste. Bevorzugte C₁-C₅₀-Alkylgruppen sind Methyl, Ethyl, Propyl, Isopropyl und Butyl.

Der Begriff "C₅-C₂₀-Cycloalkyl" umfasst u.a. die Gruppen Cyclopropyl, Cyclopentyl, Cyclohexyl, Cycloheptyl und höhergliedrige Ringe.

Der Begriff "C₅-C₂₀-Aryl", wie hierin verwendet, bezeichnet aromatische Kohlenwasserstoffe, beispielsweise Phenyl, Benzyl, Naphthyl, oder Anthryl.

Der Begriff "C₂-C₂₀-Heterocyclus" bezeichnet fakultativ substituierte Ringe mit 2-20-C-Atomen, die 1 bis 4 Heteroatome, wie etwa Sauerstoff, Schwefel und/oder Stickstoff, insbesondere Stickstoff, entweder alleine oder in Zusammenhang mit Schwefel- oder Sauerstoffringatomen haben. Diese Ringe können gesättigt oder vollständig ungesättigt oder teilweise ungesättigt sein, wobei vollständig gesättigte Ringe bevorzugt werden. Bevorzugte heterozyklische Ringe schließen Piperidinyl, Morpholinyl, Piperazinyl, 2-Amino-Imidazoyl, Tetrahydrofurano, Pyrrolo, Tetrahydrothiophenyl ein.

Der Begriff "C₂-C₂₀-Alkenyl" bezeichnet einen Rest umfassend eine Doppelbindung, wobei dieser substituiert oder unsubstituiert vorliegen kann. Die Stereolsomerie ist nicht wesentlich und alle Stereoisomere können für ein jeweiliges substituiertes Alkenyl verwendet werden.

Der Begriff "C₂-C₁₂-Alkinyl", wie hier verwendet, bezeichnet einen Rest der Formel C₂-C₁₂-C≡C-. Beispiele für C₂-C₁₂-Alkinyle schließen ein: Ethinyl, Propinyl oder Propargyl, 2-Butinyl, 2-Pentinyl, 3-Pentinyl, 2-Hexinyl, 3-Hexinyl, 4-Hexinyl, 2-Heptinyl, 3-Heptinyl, 4-Heptinyl, 5-Heptinyl, sowie Octinyl, Noninyl, Decinyl, Undecinyl, Dodecinyl, sowie Di- und Tri-ine von geraden und verzweigten Alkylketten. Bevorzugt sind solche Alkinylreste, bei denen die Dreifachbindung endständig ist.

Der Begriff "substituiert", in Verwendung mit "Alkyl", "Alkenyl" etc., bezeichnet die Substitution eines oder mehrerer Atome, in der Regel H-Atome, durch einen oder mehrere der folgenden Substituenten, bevorzugt durch einen oder zwei der folgenden Substituenten: Halogen, Hydroxy, geschütztes Hydroxy, Oxo, geschütztes Oxo, C₃-C₇-Cycloalkyl, Phenyl, Naphtyl, Amino, geschütztes Amino, monosubstituiertes Amino, geschütztes monosubstituiertes Amino, disubstituiertes Amino. Weitere Substituenten sind generell denkabr. Die substituierten Alkygruppen, Arylgruppen, Alkenylgruppen, können einmal oder mehrfach substituiert sein und bevorzugt 1- oder 2-mal, mit denselben oder unterschiedlichen Substituenten.

In dem erfindungsgemäßen Verfahren werden auch beliebige Mischungen verschiedener Triazine der Formel IV als Ausgangsverbindungen eingesetzt und umgesetzt. Es werden somit die entsprechenden Mischungen der Triazincarbamate (I) erhalten.

Die ablaufende Reaktion ist beispielhaft in der folgenden Reaktionsgleichung dargestellt:

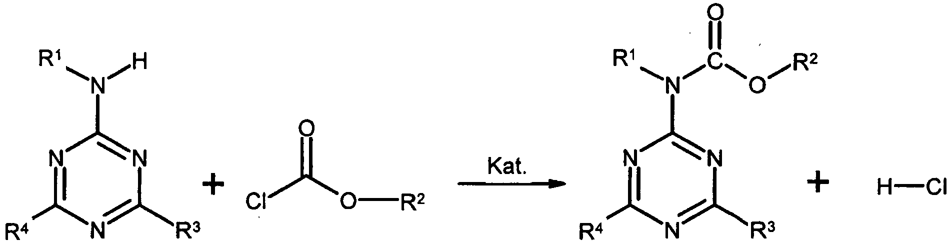

Die eingesetzte Alkali- oder Erdalkalimetallverbindung kann neben ihrer Wirkung als Aktivator idealerweise auch dazu genutzt werden, um das frei werdende HCl in Form eines Alkali- oder Erdalkalimetallchlorid zu binden.

Wie oben erwähnt werden im erfindungsgemäßen Verfahren Alkali- oder Erdalkalimetallverbindungen aus einer Gruppe umfassend NaHCO₃, KHCO₃, Na₂CO₃, K₂CO₃, MgCO₃, CaCO₃, Na₃PO₄, Na₂HPO₄, Na-acetat, Dinatriumoxalat, Butyllithium, Methyllithium, Phenyllithium, Methylnatrium, Butylnatrium, Phenylnatrium, Methylmagnesiumbromid, LiAlH₄, Natriumamid eingesetzt, wobei bevorzugt NaHCO₃, Na₂CO₃ und Butyllithium verwendet werden.

Die Alkali- und Erdalkaliverbindungen werden in einer Menge von 0,05 bis 1,2 mol Äquivalente bezogen auf die umzusetzenden HN-Gruppen eingesetzt. Bevorzugt werden dabei 0,5 bis 1,2 mol Äquivalente an Alkali- und Erdalkaliverbindungen, insbesondere bevorzugt 0,8 bis 1,2 mol Äquivalente an Alkali- und Erdalkaliverbindungen, eingesetzt.

Ein Einsatz der Alkali- oder Erdalkalimetallverbindung in stöchiometrischen Mengen oder in leichtem Überschuss, bezogen auf die umzusetzenden NH-Gruppen, gewährleistet einen raschen Fortgang der Reaktion.

Gegebenenfalls wird der Reaktionsmischung vorteilhafterweise ein weiterer Stoff zugesetzt, der das frei werdende HCl bindet und so den Reaktionsfortgang weiter beschleunigt.

dabei von Vorteil, wenn der weitere Stoff in Mengen von 0,5 bis 1,5 mol Äquivalente pro umzusetzende NH-Gruppen eingesetzt wird.

Im erfindungsgemäßen Verfahren werden je Moläquivalent NH-Gruppen im Triazin der Formel IV 0,7 bis 10,0 Mol, bevorzugt 0,9 bis 7,0 Mol an Chlorformiat der Formel V oder der Formel VI verwendet.

Das Verfahren wird vorteilhafterweise in Substanz, wobei das Chlorformiat der Formel V oder der Formel VI auch als Lösungsmittel fungiert, und/oder in einem anderen geeigneten Lösungsmittel durchgeführt werden. Bevorzugt wird die Reaktion in Lösung durchgeführt.

Geeignete Lösungsmittel zur Durchführung des erfindungsgemäßen Verfahren sind Tetrahydrofuran, Diethylether, Dimethoxymethan, Dimethoxyethan, Diethoxymethan, Diethoxyethan, Ethylenglykoldimethylether, Ethylenglykoldiethylether, Ethylenglykoldibutylether, Diethylenglykoldiethylether, Dioxan, Aceton, Methylenchlorid, Chloroform, Benzen, Toluen, Xylen, Mesitylen, Cumen, Chlorbenzen, Pentan, Hexan, Cyclohexan, Heptan, Octan, Acetonitril, Methylacetat, Ethylacetat, Methylbenzoat, N,N-Dimethylformamid, N,N-Dimethylacetamid, N-Methylpyrrolidon, 1,3-Dimethylimidazolidinon sowie die dem Fachmann als ionische Flüssigkeiten bekannten Lösungsmittel.

Das erfindungsgemäße Verfahren wird üblicherweise bei einer Temperatur von 0 bis 200 °C, durchgeführt. Als besonders vorteilhaft hat es sich erwiesen, wenn während der Reaktion ein Temperaturprofil durchlaufen wird.

Idealerweise wird die Reaktion bei tiefen Temperaturen begonnen und dann langsam bis zur gewünschten Endtemperatur erhöht. Die Reaktion wird vorteilhafterweise bei Temperaturen zwischen 0°C und 20°C (Raumtemperatur), gestartet wird und dann bis zu einer ausgewählten Endtemperatur zwischen 20°C bis 200°C, erhöht. Höhere Reaktionstemperaturen ermöglichen dabei eine Doppelsubstitution an einer NH₂-Gruppe des eingesetzten Triazins.

Vorteilhafterweise wird die erfindungsgemäße Reaktion so durchgeführt, dass die Triazinverbindung IV im geeigneten Lösungsmittel vorgelegt wird, mit der Alkali- oder Erdalkalimetallverbindung bei tiefer Temperatur versetzt wird und anschließend das Chlorformiat zugegeben wird. Anschließend erfolgt vorteilhafterweise eine Erhöhung der Temperatur zur Vervollständigung der Reaktion. Die Isolierung und Reinigung des Reaktionsproduktes erfolgt nach dem Fachmann bekannten Verfahren.

Vorteilhafterweise werden als Chlorformiate Methylchlorformiat, Butylchlorformiat, Phenylchlorformiat, Benzylchlorformiat, Menthylchlorformiat, 1-Chlorethylchlorformiat, 1-Naphthylchlorformiat, 2-Chlorethylchlorformiat, 2-Chlorbenzylchlorformiat, 2-Chlorphenylchlorformiat, 2-Ethylhexylchlorformiat, 2-Fluorethylchlorformiat, 2-Methoxyethylchlorformiat, 2-Methoxyphenylchlorformiat, 2-Nitrophenylchlorformiat, 2-Chlorpropylchlorformiat, 4-Chlorbutylchlorformiat, Allylchlorformiat, Cetylchlorformiat, Ethylchlorformiat, Ethylen-bis(chlorformiat), Hexylchlorformiat, Isobutylchlorformiat, Isopropenylchlorformiat, Neopentylchlorformiat, Octylchlorformiat, Tolylchlorformiat, Propargylchlorformiat, Propylchlorformiat, Vinylchlorformiat, 1,4-Butandiol-bis(chlorformiat), 2-Butyn-1-ylchlorformiat, 3-Butyn-1-ylchlorformiat, Bisphenol-A-bis(chlorformiat), Bisphenol-Z-bis(chlorformiat), Triethylenglycol-bis(chlorformiat) und/oder 1,4-Phenylen-bis(chlorformiat) verwendet.

Bevorzugt verwendete Chlorformiate sind Methylchlorformiat, Butylchlorformiat, Phenylchlorformiat, Allylchlorformiat, Ethylen-bis(chlorformiat), Isobutylchlorformiat, Vinylchlorformiat, 1,4-Butandiol-bis(chlorformiat), Bisphenol-A-bis(chlorformiat), Chlorpropylformiat und Propargylformiat.

Die nach dem erfindungsgemäßen Verfahren gebildeten Triazincarbamate I können vorteilhafterweise unter Abspaltung der Hydroxylverbindung R²-OH mit einer weiteren Triazinverbindung IV reagieren, so dass mehrkernige Triazinverbindungen, insbesondere 2-bis 20- kernige Triazinverbindungen, entstehen. Diese Reaktion entspricht im Prinzip einer Umesterung bzw. einer Umamidierung. Letztlich wird so aus der Carbamatverbindung eine Harnstoffbindung aufgebaut, wodurch zwei Triazinkeme miteinander verknüpft werden.

Da diese Reaktion bei Vorhandensein der geeigneten Gruppen auch mehrmals ablaufen kann, ist so der Aufbau von mehrkernigen Triazinkomplexen möglich. Die Häufigkeit dieser Reaktion kann durch die Wahl der Reaktionsbedingungen gezielt gesteuert werden.

Auch über eine Variation der Dosierreihenfolge und der Dosiergeschwindigkeit ist es möglich, die Häufigkeit der Bildung von Mehrkemverbindungen zu steuern. So wird üblicherweise das Chlorformiat zu einer Mischung aus Triazin und Alkali- oder Erdalkalimetallverbindung zugegeben. Erfolgt diese Zugabe rasch, so kommt es kaum zur Bildung von Mehrkemverbindungen. Erfolgt die Zugabe hingegen langsam, so kommt es verstärkt zur oben beschriebenen Reaktion und damit zur Bildung von Mehrkemverbindungen.

Bei den nach dem erfindungsgemäßen Verfahren erhältlichen Triazincarbamaten handelt es sich um wertvolle Rohstoffe z.B. für die Lackindustrie. Die Triazincarbamate können sowohl als Vernetzer als auch zum Aufbau von Bindemitteln z.B. in Polyester-, Polyurethan- oder Epoxybasierten Lacken verwendet werden.

Die Erfindung wird nachfolgend an mehreren Ausführungsbeispielen erörtert.

### Beispiel 1:

In einem Reaktor ausgestattet mit Rührer, Rückflusskühler und Stickstoffeinlass werden 100g N,N',N"-Trimethylmelamin, 200g Natriumhydrogencarbonat, und 1,0 l Methylchlorformiat in 1,5 l trockenem Dichlormethan vorgelegt und langsam bis zum Rückfluss erhitzt. Während der gesamten Reaktion wird ein leichter Stickstoffstrom im System aufrechterhalten. Nach 7,5 h wird auf Raumtemperatur abgekühlt und es werden 200g Natriumsulfat zugegeben. Anschließend wird die Mischung filtriert und das Filtrat wird am Rotationsverdampfer eingeengt und über Nacht im Vakuum getrocknet. Man erhält 192 g eines farblosen Feststoffes, der zu 96,8% aus N,N',N"-Tri-(methylcarbamoyl)-N,N',N"-trimethylmelamin besteht.

### Beispiel 2:

In einem Schlenkkolben werden 1,0g N,N',N"-Trimethylmelamin und 2,0g Natriumhydrogencarbonat in 10 ml trockenem THF vorgelegt und anschließend werden bei Raumtemperatur 5 ml Methylchlorformiat zugegeben und gerührt. Nach 40 h werden 2,0 g Natriumsulfat zugegeben, filtriert und das Filtrat eingeengt und im Vakuum getrocknet. Man erhält 1,6 g eines weißen Feststoffes, der zu 18% aus N,N'-Di-(methylcarbamoyl)-N,N',N"-trimethylmelamin und zu 81% aus N,N',N"-Tri-(methylcarbamoyl)-N,N',N"-trimethylmelamin besteht.

### Beispiel 3:

In einem Schlenkkolben werden 1,0g N,N',N"-Trimethylmelamin und 2,0g Natriumhydrogencarbonat in 15 ml trockenem Ethylacetat vorgelegt und anschließend werden bei Raumtemperatur 5 ml Methylchlorformiat zugegeben und gerührt. Nach 15 h werden 2,0 g Natriumsulfat zugegeben, filtriert und das Filtrat eingeengt und im Vakuum getrocknet. Man erhält 1,3 g eines weißen Feststoffes, der zu 23% aus N-(Methylcarbamoyl)-N,N',N"-trimethylmelamin, 32% aus N,N'-Di-(methylcarbamoyl)-N,N',N"-trimethylmelamin und zu 43% aus N,N',N",Tri-(methylcarbamoyl)-N,N',N"-trimethylmelamin besteht.

### Beispiel 4:

In einem 250ml Dreihalskoben ausgestattet mit Rührer, Rückflusskühler und Stickstoffeinlass werden 5,0 g (29,7 mmol) N,N',N"-Trimethylmelamin und 10,0 g (119 mmol) Natriumhydrogencarbonat in 75 ml trockenem Methylenchlorid vorgelegt und auf 30°C erwärmt. Anschließend werden 20 ml (259 mmol) Methylchlorformiat zugetropft. Daraufhin wird bei 30°C gerührt und die Zunahme an gebildetem N,N',N"-Tri-(methylcarbamoyl)-N,N',N"-trimethylmelamin mittels GC/FID bestimmt. Der folgenden Tabelle 1 sind die Werte zu entnehmen.

**Tabelle 1: Bildung von N,N',N"-Tri-(methylcarbamoyl)-N,N',N"-trimethylmelamin in Abhängigkeit von der Reaktionszeit**

| Reaktionszeit/ h | Produktgehalt/ m% |
|---|---|
| **3** | **18,5** |
| **20** | **54** |
| **25,5** | **69** |
| **44** | **76** |

### Beispiel 5:

In einem 250ml Dreihalskoben ausgestattet mit Rührer, Rückflusskühler und Stickstoffeinlass werden 5,0 g (29,7 mmol) N,N',N"-Trimethylmelamin und 10,0 g (119 mmol) Natriumhydrogencarbonat in 75 ml trockenem Methylenchlorid vorgelegt. Anschließend werden 20 ml (259 mmol) Methylchlorformiat bei Raumtemperatur zugetropft. Daraufhin wird auf 40°C erwärmt. Nach 3h wird auf Raumtemperatur abgekühlt und es werden 10g Natriumsulfat zugegeben. Anschließend wird die Mischung filtriert und das Filtrat wird am Rotationsverdampfer eingeengt und über Nacht im Vakuum getrocknet. Man erhält 16,3 g eines farblosen Feststoffes der aus 7% N-(Methylcarbamoyl)-N,N',N"-trimethylmelamin, 38% N,N'-Di-(methylcarbamoyl)-N,N',N"-trimethylmelamin und 55% N,N',N"-Tri-(methylcarbamoyl)-N,N',N"-trimethylmelamin besteht.

### Beispiel 6:

In einem 250ml Dreihalskoben ausgestattet mit Rührer, Rückflusskühler und Stickstoffeinlass werden 5,0 g (29,7 mmol) N,N',N"-Trimethylmelamin und 10,0 g (119 mmol) Natriumhydrogencarbonat in 75 ml trockenem Methylenchlorid vorgelegt. Anschließend werden 50 ml (648 mmol) Methylchlorformiat bei Raumtemperatur zugetropft. Daraufhin wird auf 40°C erwärmt. Nach 3h wird auf Raumtemperatur abgekühlt und es werden 10g Natriumsulfat zugegeben. Anschließend wird die Mischung filtriert und das Filtrat wird am Rotationsverdampfer eingeengt und über Nacht im Vakuum getrocknet. Man erhält 18,4 g eines farblosen Feststoffes der aus 21% N,N'-Di-(methylcarbamoyl)-N,N',N"-trimethylmelamin und 79% N,N',N"-Tri-(methylcarbamoyl)-N,N',N"-trimethylmelamin besteht.

### Beispiel 7:

In einem 500 ml Rundkolben werden 15,0 g (0,09 mol) N,N',N"-Trimethylmelamin in 240 ml Dioxan aufgelöst und auf 10°C gekühlt. Dann werden bei 10°C 157 ml (0,283 mol) n-Butyllithium (1,6 molar in Hexan) zugetropft. Es wird eine Stunde gerührt. Anschließend werden 30 ml (0,389 mol) Methylchlorformiat in 30 ml Dioxan unter intensiver Kühlung in einem Zeitraum von 15 min rasch zugetropft. Nach beendeter Zugabe wird auf Raumtemperatur erwärmen gelassen und weitere 20 h gerührt. Dann wird die Reaktionsmischung in 600 ml Salzsäure (pH = 2) gegossen, wobei sich eine klare Lösung bildet. Mit NaOH-Lösung wird ein pH-Wert von 6 eingestellt und ca. 1 h gerührt, um überschüssiges Methylchlorformiat zu zersetzen. Dann wird die Lösung weitgehend eingeengt und der sich dabei bildende Niederschlag abfiltriert. Nach Trocknung des Niederschlages erhält man 27,3 g weißen Feststoff der aus 1,8 % N,N'-Di-(methylcarbamoyl)-N,N',N"-trimethylmelamin, 79,5% N,N',N"-Tri-(methylarbamoyl)-N,N',N"-trimethylmelamin, 13,4 % der unten abgebildeten zweikernigen Spezies der Formel (VII) und 4,2% analogen drei- und vierkernigen Spezies besteht.

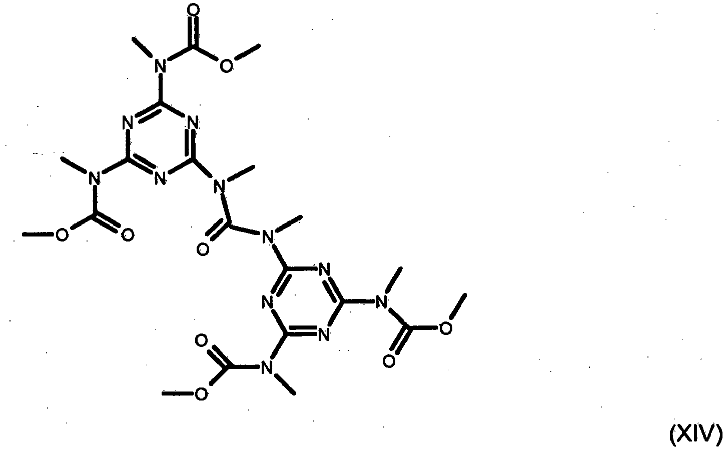

### Beispiel 8

In einem 500 ml Rundkolben werden 15,0 g (0,09 mol) N,N',N"-Trimethylmelamin in 240 ml Dioxan aufgelöst und auf 10°C gekühlt. Dann werden bei 10°C 157 ml (0,283 mol) n-Butyllithium (1,6 molar in Hexan) zugetropft. Es wird eine Stunde gerührt. Anschließend werden 30 ml (0,389 mol) Methylchlorformiat in 30 ml Dioxan über einen Zeitraum von 45 min langsam zugetropft. Nach beendeter Zugabe wird auf Raumtemperatur erwärmen gelassen und weitere 20 h gerührt. Dann wird die Reaktionsmischung in 600 ml Salzsäure (pH = 2) gegossen wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 29,2 g weißen Feststoff der aus 0,1 % N,N'-Di-(methylcarbamoyl)-N,N',N"-trimethylmelamin, 17,1% N,N',N"-Tri-(methylcarbamoyl)-N,N',N"-trimethylmelamin, 17,9 % der abgebildeten zweikernigen Spezies der Formel (XIV) und 60,3% analogen drei- und vierkernigen Spezies besteht.

### Beispiel 9:

In einem 250 ml Rundkolben werden 5,0 g (0,03 mol) N,N',N"-Trimethylmelamin in 80 ml THF aufgelöst und auf 0°C gekühlt. Dann werden bei 0°C 59 ml (0,106 mol) n-Butyllithium (1,6 molar in Hexan) zugetropft. Es wird eine Stunde gerührt. Anschließend werden 14,5 ml (0,188 mol) Methylchlorformiat in 15 ml THF unter intensiver Kühlung zugetropft. Nach beendeter Zugabe wird noch 2 h bei 0°C gerührt und dann auf Raumtemperatur erwärmen gelassen und weitere 20 h gerührt. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 9,7 g weißen Feststoff der aus 2,3 % N,N'-Di-(methylcarbamoyl)-N,N',N"-trimethylmelamin, 85,2% N,N',N"-Tri-(methylcarbamoyl)-N,N',N"-trimethylmelamin und 12,5 % der zweikernigen Spezies der Formel (XIV) besteht.

### Beispiel 10:

In einem 250 ml Rundkolben werden 5,0 g (0,03 mol) N,N',N"-Trimethylmelamin in 80 ml Dioxan aufgelöst und auf 10°C gekühlt. Dann werden bei 10°C 84 ml (0,151 mol) n-Butyllithium (1,6 molar in Hexan) zugetropft. Es wird eine Stunde gerührt. Anschließend werden 20,7 ml (0,268 mol) Methylchlorformiat in 20 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 10,6 g weißen Feststoff der aus 5,6 % N,N'-Di-(methylcarbamoyl)-N,N',N"-trimethylmelamin, 78,8% N,N',N"-Tri-(methylcarbamoyl)-N,N',N"-trimethylmelamin und 15,6 % der zweikernigen Spezies VI besteht.

### Beispiel 11:

In einem 250 ml Rundkolben werden 5,0 g (0,03 mol) N,N',N"-Trimethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 14,5 ml (0,188 mol) Methylchlorformiat in 15 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 10,6 g weißen Feststoff der aus 2,8 % N,N'-Di-(methylcarbamoyl)-N,N',N"-trimethylmelamin, 87,5% N,N',N"-Tri-(methylcarbamoyl)-N,N',N"-trimethylmelamin und 9,7 % der zweikernigen Spezies VI besteht.

### Beispiel 12:

In einem 250 ml Rundkolben werden 4,62 g (0,03 mol) N,N'-Dimethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 5,1 ml (0,066 mol) Methylchlorformiat in 10 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 6,7 g weißen Feststoff der aus 62,4 % N-(Methylcarbamoyl)-N,N'-dimethylmelamin, 23,9% N,N'-Di-(methylcarbamoyl)-N,N'-dimethylmelamin und 7,3 % N,N',N"-Tri-(methylcarbamoyl)-N,N'-dimethylmelamin besteht.

### Beispiel 13:

In einem 250 ml Rundkolben werden 3,78 g (0,03 mol) Melamin in 80 ml Dioxan suspendiert und auf 10°C gekühlt. Dann werden bei 10°C 59 ml (0,106 mol) n-Butyllithium (1,6 molar in Hexan) zugetropft. Es wird eine Stunde gerührt. Anschließend werden 8,05 ml (0,104 mol) Methylchlorformiat in 12 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Im Verlauf der Zeit bildet sich eine klare Lösung. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt, um überschüssiges Methylchlorformiat zu zersetzen. Dann wird die Lösung weitgehend eingeengt und der sich dabei bildende Niederschlag abfiltriert. Nach Trocknung des Niederschlages erhält man 3,3 g weißen Feststoff, der aus 69 % N-(Methylcarbamoyl)-melamin, 25,7% N,N'-Di-(methylcarbamoyl)-melamin und 5,3 % N,N',N"-Tri-(methylcarbamoyl)-melamin besteht.

### Beispiel 14:

In einem 250 ml Rundkolben werden 5,0 g (0,03 mol) N,N',N"-Trimethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 23,9 ml (0,188 mol) Butylchlorformiat in 25 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen wobei sich 2 Phasen bilden. Mit NaOH-Lösung wird ein pH-Wert von 6 eingestellt und ca. 1 h gerührt um überschüssiges Butylchlorformiat zu zersetzen. Dann wird die Lösung mit 3x mit Methylenchlorid extrahiert und die organische Phase zur Gänze eingeengt. Man erhält 15,8 g einer klaren Flüssigkeit der aus 8,4 % N,N'-Di-(butylcarbamoyl)-N,N',N"-trimethylmelamin und 90,4 % N,N',N"-Tri-(butylcarbamoyl)-N,N',N"-trimethylmelamin besteht.

### Beispiel 15:

In einem 250 ml Rundkolben werden 5,0 g (0,03 mol) N,N',N"-Trimethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 23,2 g (0,108 mol) 1,4-Butandiol-bis(chlorformiat) in 25 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen wobei sich eine klare Lösung bildet. Mit NaOH-Lösung wird ein pH-Wert von 6 eingestellt und ca. 1 h gerührt um überschüssiges Butylchlorformiat zu zersetzen. Dann wird die Lösung weitgehend eingeengt und der sich dabei bildende Niederschlag abfiltriert. Nach Trocknung des Niederschlages erhält man 15,8 g weißen Feststoff der aus 74,7 % der zweikernigen Spezies nach Abbildung XV und 18,4 % entsprechenden dreikernigen Spezies sowie 6,2 % vierkernigen Spezies.

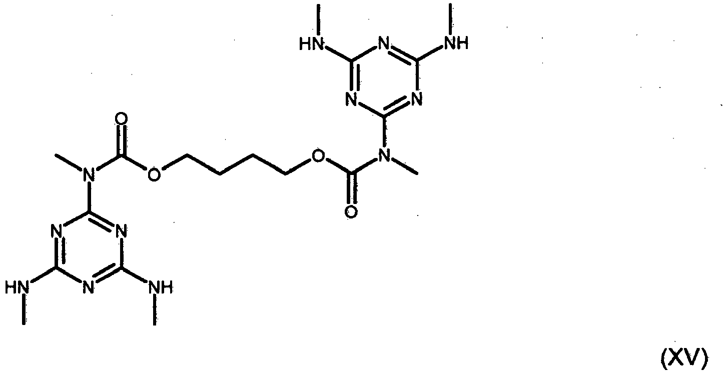

### Beispiel 16:

In einem 250 ml Rundkolben werden 5,0 g (0,027 mol) N,N,N',N"-Tetramethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 15,4 ml (0,20 mol) Methylchlorformiat in 20 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 8,4 g weißen Feststoff der aus 18,4 % N'-(Methylcarbamoyl)-N,N,N',N"-tetramethylmelamin und 80,6 % N',N"-Di-(methylcarbamoyl)-N,N,N',N"-tetramethylmelamin besteht.

### Beispiel 17:

In einem 250 ml Rundkolben werden 4,62 g (0,03 mol) N,N-Dimethylmelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 5,1 ml (0,066 mol) Methylchlorformiat in 10 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 6,9 g weißen Feststoff der aus 40,4 % N'-(Methylcarbamoyl)-N,N-dimethylmelamin, 47,8 % N',N"-Di-(methylcarbamoyl)-N,N-dimethylmelamin und 10,4 % N',N',N"-Tri-(methylcarbamoyl)-N,N-dimethylmelamin besteht.

### Beispiel 18:

In einem 250 ml Rundkolben werden 6,3 g (0,03 mol) Succinimidomelamin in 80 ml Dioxan aufgelöst und auf 10°C gekühlt. Dann werden bei 10°C 59 ml (0,106 mol) n-Butyllithium (1,6 molar in Hexan) zugetropft. Es wird eine Stunde gerührt. Anschließend werden 5,1 ml (0,066 mol) Methylchlorformiat in 10 ml Dioxan unter intensiver Kühlung zugetropft. Nach beendeter Zugabe wird noch 2 h bei 10°C gerührt und dann auf Raumtemperatur erwärmen gelassen und weitere 20 h gerührt. Dann wird die Reaktionsmischung in 200 ml Wasser/HCl (pH = 2) gegossen, wobei sich eine klare Lösung bildet. Mit NaOH-Lösung wird ein pH-Wert von 6 eingestellt und ca. 1 h gerührt um überschüssiges Methylchlorformiat zu zersetzen. Dann wird die Lösung weitgehend eingeengt und der sich dabei bildende Niederschlag abfiltriert. Nach Trocknung des Niederschlages erhält man 7,9 g weißen Feststoff der aus 28,2 % N'-(Methylcarbamoyl)-succinimidomelamin, 65,4 % N',N"-Di-(methylcarbamoyl)-succinimidomelamin und 5,9 % N',N',N"-Tri-(methylcarbamoyl)-succinimidomelamin besteht.

## Patentansprüche

1. Verfahren zur Herstellung von mindestens einem Triazincarbamat der Formel I 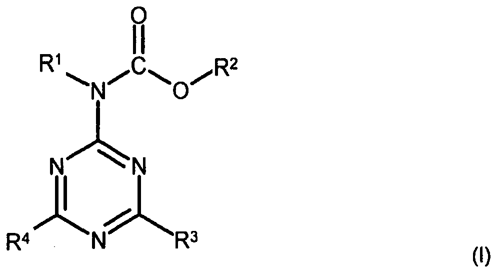 oder deren Mischungen, wobei
**R³** einen mit seinem zentralen Stickstoffatom an ein C-Atom des Triazinringes der Struktur der Formel (I) gebundenen Rest der Formel **R⁵-N-R⁶** bedeutet,
**R⁴** einen mit dem Stickstoffatom an ein C-Atom des Triazinringes der Struktur der Formel (I) gebundenen Rest der Formel **R⁷**-N**-R⁸** bedeutet,
**R⁶** und **R⁸** unabhängig voneinander H, Q², -CO-O-**R²**, -CO-**R⁹** oder -CO-O-**R¹⁰** und
**R^{1,} R⁵** und **R⁷** unabhängig voneinander Q², -CO-O-**R²**, -CO-**R⁹** oder -CO-O-**R¹⁰** bedeuten, wobei
- Q² jeweils ein lineares oder verzweigtes C₁-C₅₀-Alkyl, C₅-C₂₀-Cycloalkyl, C₅-C₂₀-Aryl, C₁-C₅₀-alkylsubstitutiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₂₀-Alkenyl, C₂-C₁₂-Alkinyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH- und/oder -OC(O)O- unterbrochen sein kann, ist,
**R²** ein lineares oder verzweigtes C₁-C₅₀-Alkyl, C₅-C₂₀-Cycloalkyl, C₅-C₂₀-Aryl, C₁-C₅₀-alkylsubstituiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₁₂-Alkinyl, C₂-C₂₀-Alkenyl oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH- und/oder -OC(O)O- unterbrochen sein kann und/oder ein oder mehrere Halogenatome und/oder Nitrogruppen als Substituenten aufweist, ist,
**R⁹** einen Rest der allgemeinen Formel (II) 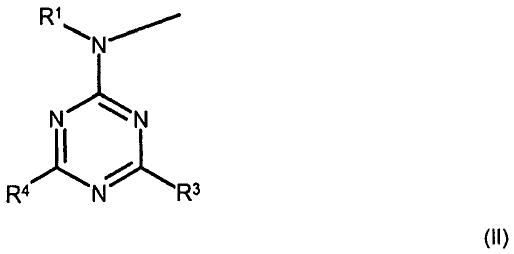 bedeutet,
**R¹⁰** einen Rest der allgemeinen Formel (III) 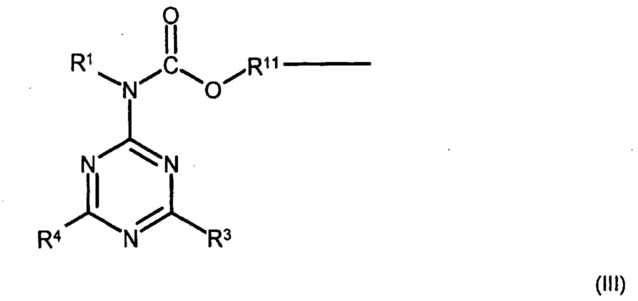 bedeutet, wobei
- **R¹¹** jeweils ein lineares oder verzweigtes C₁-C₅₀-Alkyl, C₅-C₂₀-Cycloalkyl, C₅-C₂₀-Aryl, C₁-C₅₀-alkylsubstituiertes C₅-C₂₀-Aryl, C₂-C₂₀-Heterocyclus, C₂-C₂₀-alkenylsubstituiertes C₂-C₂₀-Heterocyclus, C₁-C₅₀-Alkylsubstituiertes C₂-C₂₀-Heterocyclus, C₂-C₂₀-Alkenyl, C₂-C₁₂-Alkinyl, oder C₂-C₂₀-alkenylsubstituiertes C₅-C₂₀-Aryl, das jeweils durch ein oder mehrere Sauerstoffatome, Schwefelatome, substituierte und/oder unsubstituierte Stickstoffatome, durch Doppelbindungen, Siloxangruppen und/oder durch ein oder mehrere Gruppen des Typs -C(O)O-, -OC(O)-, -C(O)-, -NHC(O)O-, -OC(O)NH- und/oder -OC(O)O- unterbrochen sein kann, ist,
**gekennzeichnet durch**
die Umsetzung von mindestens einem Triazin der Formel IV 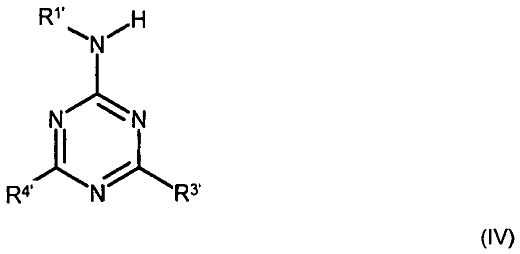 wobei R^{1'} die Bedeutung von R¹, R^{3'} die Bedeutung von R³ und R^{4'} die Bedeutung von R⁴ aufweist
mit mindestens einem Chlorformiat der allgemeinen Formel (V) 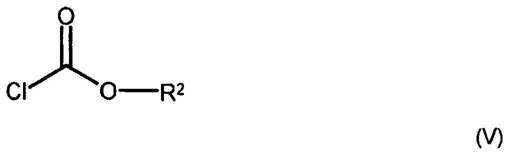 und/oder der allgemeinen Formel (VI) 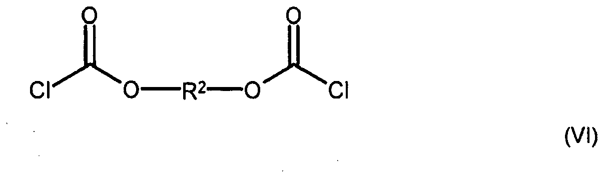 in Gegenwart mindestens einer Alkali- oder Erdalkalimetallverbindung aus der Gruppe umfassend NaHCO₃, KHCO₃, Na₂CO₃, K₂CO₃, MgCO₃, CaCO₃, Na₃PO₄, Na₂HPO₄, Naacetat, Dinatriumoxalat, Butyllithium, Methyllithium, Phenyllithium, Methylnatrium, Butylnatrium, Phenylnatrium, Methylmagnesiumbromid, LiAlH₄, Natriumamid verwendet wird, und wobei die Alkali- oder Erdalkalimetallverbindung nicht in Form eines Alkoholates vorliegt

2. Verfahren nach Anspruch 1, **dadurch gekennzeichnet, dass** als Chlorformiat Methylchlorformiat, Butylchlorformiat, Phenylchlorformiat, Benzylchlorformiat, Menthylchlorformiat, 1-Chlorethylchlorformiat, 1-Naphthylchlorformiat, 2-Chlorethylchlorformiat, 2-Chlorbenzylchlorformiat, 2-Chlorphenylchlorformiat, 2-Ethylhexylchlorformiat, 2-Flourethylchlorformiat, 2-Methoxyethylchlorformiat, 2-Methoxyphenylchlorformiat, 2-Nitrophenylchlorformiat, 2-Chlorpropylchlorformiat, 4-Chlorbutylchlorformiat, Allylchlorformiat, Cetylchlorformiat, Ethylchlorformiat, Ethylen-bis(chlorformiat), Hexylchlorformiat, Isobutylchlorformiat, Isopropenylchlorformiat, Neopentylchlorformiat, Octylchlorformiat, Tolylchlorformiat, Propargylchlorformiat, Propylchlorformiat, Vinylchlorformiat, 1,4-Butandiol-bis(chlorformiat), 2-Butyn-1-ylchlorformiat, 3-Butyn-1-ylchlorformiat, Bisphenol-A-bis(chlorformiat), Bisphenol-Z-bis(chlorformiat), Triethylenglycol-bis(chlorformiat), 1,4-Phenylen-bis(chlorformiat) oder beliebige Mischungen davon eingesetzt wird.

3. Verfahren nach Anspruch 2, **dadurch gekennzeichnet, dass** als Chlorformiat Methylchlorformiat, Butylchlorformiat, Phenylchlorformiat, Allylchlorformiat, Ethylen-bis(chlorformiat), Isobutylchlorformiat, Vinylchlorformiat, 1,4-Butandiol-bis(chlorformiat), 2-Chlorpropylchlorformiat, Propargylchlorformiat oder Bisphenol-A-bis(chlorformiat) eingesetzt werden.

4. Verfahren nach einem der vorhergehenden Ansprüche, **dadurch gekennzeichnet, dass** als Alkali- oder Erdalkaliverbindung Butyllithium, NaHCO₃ oder Na₂CO₃ verwendet wird.

5. Verfahren nach einem der vorhergehenden Ansprüche, **dadurch gekennzeichnet, dass** pro NH-Gruppen des Triazins 0,05 bis 1,2 mol Äquivalente an Alkali- und Erdalkaliverbindungen, bevorzugt 0,5 bis 1,2 mol Äquivalente, insbesondere bevorzugt 0,8 bis 1,2 mol Äquivalente, eingesetzt werden.

6. Verfahren nach einem der vorhergehenden Ansprüchen, **dadurch gekennzeichnet, dass** die Reaktion bei Temperaturen von 0 bis 200°C durchgeführt wird.

7. Verfahren nach einem der vorhergehenden Ansprüchen, **dadurch gekennzeichnet, dass** während der Reaktion ein Temperaturprofil durchlaufen wird.

8. Verfahren nach Anspruch 7, **dadurch gekennzeichnet, dass** die Reaktion bei niederen Temperaturen, insbesondere bei Temperaturen zwischen 0°C und 20°C, gestartet wird und dann bis zu einer ausgewählten Endtemperatur, insbesondere Temperaturen zwischen 20°C bis 200°C, erhöht wird.

9. Verfahren nach einem der vorhergehenden Ansprüche, **dadurch gekennzeichnet, dass** pro Moläquivalent NH-Gruppen des Triazins 0,7 bis 10,0 Mol, bevorzugt 0,9 bis 7,0, Chlorformiat eingesetzt werden.

10. Verfahren nach einem der vorhergehenden Ansprüche, **dadurch gekennzeichnet, dass** die Reaktion in einem Lösungsmittel oder in Substanz, wobei das Chlorformiat gemäß Formel (V) und/oder (VI) als Lösungsmittel fungiert, durchgeführt wird.

## Claims

1. Method for producing triazine carbamate of formula I 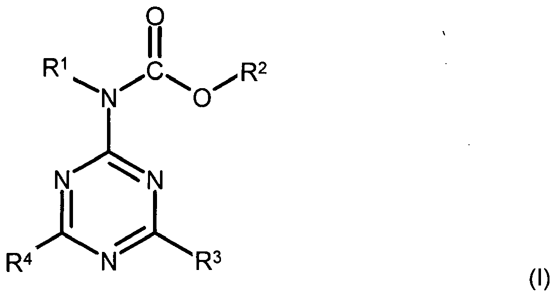 or mixtures thereof, wherein
R³ means a moiety of the formula R⁵-N-R⁶ bound with its central nitrogen atom to a C-atom of the triazine ring of the structure of formula (I),
R⁴ means a moiety of the formula R⁷-N-R⁸ bound with the nitrogen atom to a C-atom of the triazine ring of the structure of formula (I),
R⁶ and R⁸ mean independently from each other H, Q², -CO-O-R², -CO-R⁹ or -CO-O-R¹⁰ and
R¹, R⁵ and R⁷ mean independently from each other Q², -CO-O-R², -CO-R⁹ or -CO-O-R¹⁰ wherein
- Q² is in each case a linear or branched C₁-C₅₀-alkyl, C₅-C₂₀-cycloalkyl, C₅-C₂₀-aryl, C₁-C₅₀-alkyl substituted C₅-C₂₀-aryl, C₂-C₂₀-heterocycle, C₂-C₂₀-alkenyl substituted C₂-C₂₀-heterocycle, C₁-C₅₀-alkyl substituted C₂-C₂₀-heterocycle, C₂-C₂₀-alkenyl, C₂-C₁₂-alkinyl or C₂-C₂₀-alkenyl substituted C₅-C₂₀-aryl, which in each case can be interrupted by one or multiple oxygen atoms, sulphur atoms, substituted and/or unsubstituted nitrogen atoms, by double bounds, siloxane groups and/or by one or multiple groups of the type-C(O)O-, -OC(O)-, -C(O)-, -NHC(O)O-, -OC(O)NH- and/or -OC(O)O-,
R² is a linear or branched C₁-C₅₀-alkyl, C₅-C₂₀-cyclo alkyl, C₅-C₂₀-aryl, C₁-C₅₀-alkyl substituted C₅-C₂₀-aryl, C₂-C₂₀-heterocycle, C₂-C₂₀-alkenyl substituted C₂-C₂₀-heterocycle, C₁-C₅₀-alkyl substituted C₂-C₂₀-heterocycle, C₂-C₁₂-alkinyl, C₂-C₂₀-alkenyl or C₂-C₂₀-alkenyl substituted C₅-C₂₀-aryl, which in each case can be interrupted by one or multiple oxygen atoms, sulphur atoms, substituted and/or unsubstituted nitrogen atoms, by double bounds, siloxane groups and/or by one or multiple groups of the type -C(O)O-, -OC(O)-, - C(O)-, -NHC(O)O-, -OC(O)NH- and/or -OC(O)O- and/or have one or multiple halogen atoms and/or nitro groups as substituents
R⁹ means a moiety of the general formula (II) 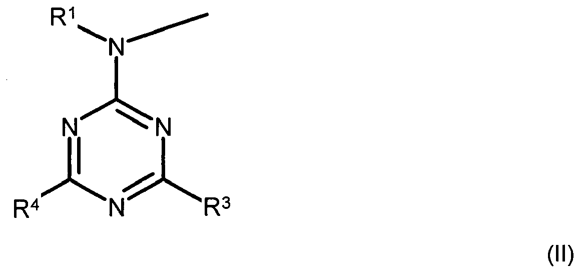 R¹⁰ means a moiety of the general formula (III) 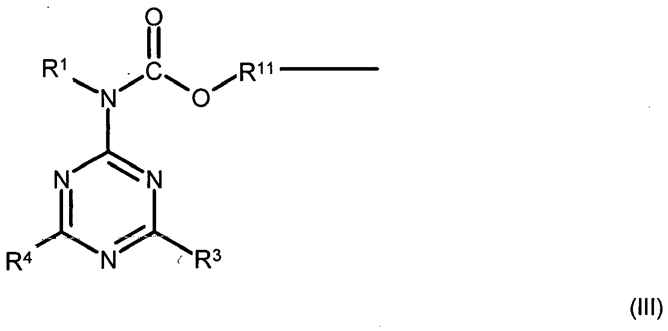 wherein
- R¹¹ is in each case a linear or branched C₁-C₅₀-alkyl, C₅-C₂₀-cycloalkyl, C₅-C₂₀-aryl, C₁-C₅₀-alkyl substituted C₅-C₂₀-aryl, C₂-C₂₀-heterocycle, C₂-C₂₀-alkenyl substituted C₂-C₂₀-heterocycle, C₁-C₅₀-alkyl substituted C₂-C₂₀-heterocycle, C₂-C₂₀-alkenyl, C₂-C₁₂-alkinyl, or C₂-C₂₀-alkenyl substituted C₅-C₂₀-aryl, which can be interrupted in each case by one or multiple oxygen atoms, sulphur atoms, substituted and/or unsubstituted nitrogen atoms, by double bounds, siloxane groups and/or by one or multiple groups of the type -C(O)O-, -OC(O)-, -C(O)-, -NHC(O)O-, -OC(O)NH- and/or -OC(O)O- , **characterized by**
the conversion of at least one triazine of the formula IV 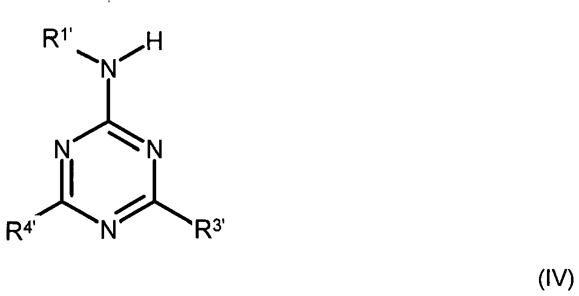 wherein R^{1'} has the meaning of R¹, R^{3'} the meaning of R³ and R^{4'} has the meaning of R4, R⁴
with at least one chloroformat of the general formula (V) 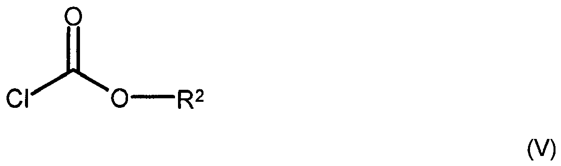 and/or the general formula (VI) 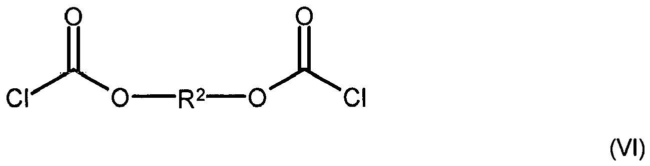 in the presence of at least one alkaline or alkaline earth metal compound from the group comprising NaHCO₃, KHCO₃, Na₂CO₃, K₂CO₃, MgCO₃, CaCO₃, Na₃PO₄, Na₂HPO₄, Na-acetate, disodium oxalate, butyl lithium, methyl lithium, phenyl lithium, methyl sodium, butyl sodium, phenyl sodium, methylmagnesium bromide, LiAlH₄, sodium amide is used, wherein the alkaline or alkaline earth metal compound is not present in form of an alcoholate.

2. Method according to claim 1, **characterized in that,** as chloroformate methylchloroformate, butylchloroformate, phenylchloroformate, benzylchloroformate, menthylchloroformate, 1-chloroethylchloroformate, 1-naphthylchloroformate, 2-chloroethylchloroformate, 2-chlorobenzylchloroformate, 2-chlorophenylchloroformate, 2-ethylhexylchloroformate, 2-fluorethylchloroformate, 2-methoxyethylchloroformate, 2-methoxyphenylchloroformate, 2-nitrophenylchloroformate, 2-chloropropylchloroformate, 4-chlorobutylchloroformate, allylchloroformate, cetylchloroformate, ethylchloroformate, ethylen-bis(chloroformate), hexylchloroformate, isobutylchloroformate, isopropenylchloroformate, neopentylchloroformate, octylchloroformate, tolylchloroformate, propargylchloroformate, propylchloroformate, vinylchloroformate, 1,4-butandiol-bis(chloroformate), 2-butyn-1-ylchloroformate, 3-butyn-1-ylchloroformate, bisphenol-A-bis(chloroformate), bisphenol-Z-bis(chloroformate), triethylenglycol-bis(chloroformate), 1,4-phenylen-bis(chloroformate) or any mixtures thereof are used.

3. Method according to claim 2, **characterized in that,** as chloroformate methylchloroformate, butychloroformate, phenylchloroformate, allylchloroformate, ethylen-bis(chloroformate), isobutylchloroformate, vinylchloroformate, 1,4-butandiol-bis(chloroformate), 2-chloropropylchloroformate, propargylchloroformate or bisphenol-A-bis(chloroformate) are used.

4. Method according to any of the preceding claims, **characterized in that,** as alkaline or alkaline earth compound butyl lithium, NaHCO₃ or Na₂CO₃ is used.

5. Method according to any of the preceding claims, **characterized in that,** per NH-groups of the triazine 0.05 to 1.2 mol equivalents of an alkaline and alkaline earth compound, preferably 0.5 to 1.2 mol equivalents, in particular preferably 0.8 to 1.2 mol equivalents are used.

6. Method according to any of the preceding claims, **characterized in that** the reaction is carried out at temperatures of 0 to 200°C.

7. Method according to any of the preceding claims, **characterized in that** a temperature profile is being conducted during the reaction.

8. Method according to claim 7, **characterized in that** the reaction is started at low temperatures, in particular at temperatures between 0°C and 20°C and is then increased to a selected final temperature, in particular temperatures between 20°C to 200°C.

9. Method according to any of the preceding claims, **characterized in that** per mol equivalent NH-groups of the triazine 0.7 to 10.0 mol, preferably 0.9 to 7.0 chloroformate are used.

10. Method according to any of the preceding claims, **characterized in that** the reaction is carried out in a solvent or in a substance, wherein the chloroformate according to formula (V) and/or (VI) acts as a solvent.

## Revendications

1. Procédé de préparation d'au moins un triazinecarbamate de formule 1 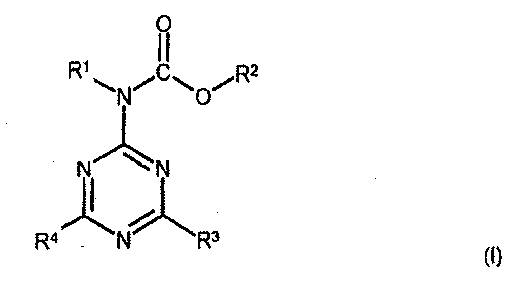 ou de leurs mélanges, dans lequel
R³ représente un radical de formule R⁵-N-R⁶, lié par son atome d'azote central à un atome de carbone du noyau triazine de la structure de formule (I),
R⁴ représente un radical de formule R⁷-N-R⁸ lié par l'atome d'azote à un atome de carbone du noyau triazine de la structure de formule (I),
R⁶ et R⁸ représentent chacun indépendamment de l'autre H, Q², -CO-O-R², -CO-R⁹ ou -CO-O-R¹⁰, et
R¹, R⁵ et R⁷ représentent chacun indépendamment des autres Q², -CO-O-R², -CO-R⁹ ou -CO-O-R¹⁰, où
chaque Q² est un radical alkyle en C₁-C₅₀ linéaire ou ramifié, cycloalkyle en C₅-C₂₀, aryle en C₅-C₂₀, aryle en C₅-C₂₀ substitué par un alkyle en C₁-C₅₀, hétérocyclyle en C₂-C₂₀, hétérocyclyle en C₂-C₂₀ substitué par un alcényle en C₂-C₂₀, hétérocyclyle en C₂-C₂₀ substitué par un alkyle en C₁-C₅₀, alcényle en C₂-C₂₀, alcynyle en C₂-C₁₂ ou aryle en C₅-C₂₀ substitué par un alcényle en C₂-C₂₀, chacun pouvant être interrompu par un ou plusieurs atomes d'oxygène, atomes de soufre, atomes d'azote substitués et/ou non substitués, par des doubles liaisons, des groupes siloxane et/ou par un ou plusieurs groupes du type -C(O)O-, -OC(O)-, -C(O)-, -NHC(O)O-, -OC(O)NH- et/ou -OC(O)O-,
R² est un radical alkyle en C₁-C₅₀ linéaire ou ramifié, cycloalkyle en C₅-C₂₀, aryle en C₅-C₂₀, aryle en C₅-C₂₀ substitué par un alkyle en C₁-C₅₀, hétérocyclyle en C₂-C₂₀, hétérocyclyle en C₂-C₂₀ substitué par un alcényle en C₂-C₂₀, hétérocyclyle en C₂-C₂₀ substitué par un alkyle en C₁-C₅₀, alcynyle en C₂-C₁₂, alcényle en C₂-C₂₀ ou aryle en C₅-C₂₀ substitué par un alcényle en C₂-C₂₀, chacun pouvant être interrompu par un ou plusieurs atomes d'oxygène, atomes de soufre, atomes d'azote substitués et/ou non substitués, par des doubles liaisons, des groupes siloxane et/ou par un ou plusieurs groupes du type -C(O)O-, -OC(O)-, -C(O)-, -NHC(O)O-, -OC(O)NH- et/ou -OC(O)O-, et/ou comprend un ou plusieurs atomes d'halogène et/ou groupes nitro en tant que substituants,
R⁹ représente un radical de formule générale (II) 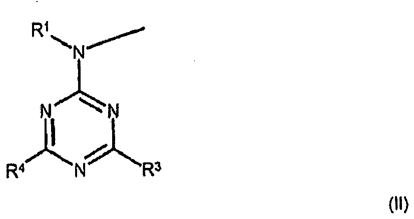 R¹⁰ représente un radical de formule générale (III) 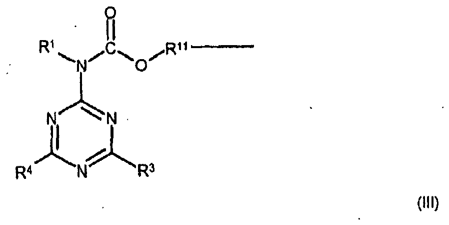 où
chaque R¹¹ est un radical alkyle en C₁-C₅₀ linéaire ou ramifié, cycloalkyle en C₅-C₂₀, aryle en C₅-C₂₀, aryle en C₅-C₂₀ substitué par un alkyle en C₁-C₅₀, hétérocyclyle en C₂-C₂₀, hétérocyclyle en C₂-C₂₀ substitué par un alcényle en C₂-C₂₀, hétérocyclyle en C₂-C₂₀ substitué par un alkyle en C₁-C₅₀, alcényle en C₂-C₂₀, alcynyle en C₂-C₁₂ ou aryle en C₅-C₂₀ substitué par un alcényle en C₂-C₂₀, chacun pouvant être interrompu par un ou plusieurs atomes d'oxygène, atomes de soufre, atomes d'azote substitués et/ou non substitués, par des doubles liaisons, des groupes siloxane et/ou par un ou plusieurs groupes du type -C(O)O-, -OC(O)-,-C(O)-, -NHC(O)O-, -OC(O)NH- et/ou -OC(O)O-,
**caractérisé par**
la réaction d'au moins une triazine de formule (IV) 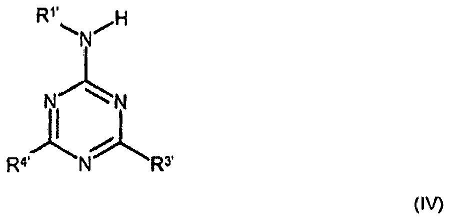 dans laquelle R^{1'} a la signification de R¹, R^{3'} a la signification de R³ et R^{4'} a la signification de R⁴,
avec au moins un chloroformiate de formule générale (V) 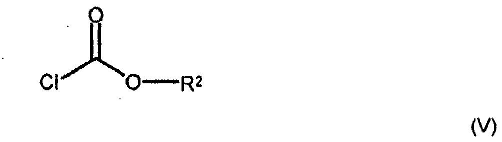 et/ou de formule générale (VI) 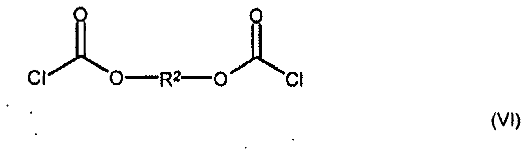 en présence d'au moins un composé d'un métal alcalin ou d'un métal alcalino-terreux du groupe comprenant NaHCO₃, KHCO₃, Na₂CO₃, K₂CO₃, MgCO₃, CaCO₃, Na₃PO₄, Na₂HPO₄, l'acétate de sodium, l'oxalate disodique, le butyllithium, le méthyllithium, le phényllithium, le méthylsodium, le butylsodium, le phénylsodium, le bromure de méthylmagnésium, LiAlH₄, l'amidure de sodium, et où le composé d'un métal alcalin ou d'un métal alcalino-terreux ne se présente pas sous la forme d'un alcoolate.

2. Procédé selon la revendication 1, **caractérisé en ce qu'**on utilise en tant que chloroformiate le chloroformiate de méthyle, le chloroformiate de butyle, le chloroformiate de phényle, le chloroformiate de benzyle, le chloroformiate de menthyle, le chloroformiate de 1-chloroéthyle, le chloroformiate de 1-naphtyle, le chloroformiate de 2-chloroéthyle, le chloroformiate de 2-chlorobenzyle, le chloroformiate de 2-chlorophényle, le chloroformiate de 2-éthylhexyle, le chloroformiate de 2-fluoroéthyle, le chloroformiate de 2-méthoxyéthyle, le chloroformiate de 2-méthoxyphényle, le chloroformiate de 2-nitrophényle, le chloroformiate de 2-chloropropyle, le chloroformiate de 4-chlorobutyle, le chloroformiate d'allyle, le chloroformiate de cétyle, le chloroformiate d'éthyle, le bis(chloroformiate) d'éthylène, le chloroformiate d'hexyle, le chloroformiate d'isobutyle, le chloroformiate d'isopropényle, le chloroformiate de néopentyle, le chloroformiate d'octyle, le chloroformiate de tolyle, le chloroformiate de propargyle, le chloroformiate de propyle, le chloroformiate de vinyle, le bis(chloroformiate) de 1,4-butanediol, le chloroformiate de 2-butyn-1-yle, le chloroformiate de 3-butyn-1-yle, le bis(chloroformiate) de bisphénol-A, le bis(chloroformiate) de bisphénol-Z, le bis(chloroformiate) de triéthylèneglycol, le bis(chloroformiate) de 1,4-phénylène ou les mélanges quelconques de ceux-ci.

3. Procédé selon la revendication 2, **caractérisé en ce qu'**on utilise en tant que chloroformiate le chloroformiate de méthyle, le chloroformiate de butyle, le chloroformiate de phényle, le chloroformiate d'allyle, le bis(chloroformiate) d'éthylène, le chloroformiate d'isobutyle, le chloroformiate de vinyle, le bis(chloroformiate) de 1,4-butanediol, le chloroformiate de 2-chloropropyle, le chloroformiate de propargyle ou le bis(chloroformiate) de bisphénol-A.

4. Procédé selon l'une des revendications précédentes, **caractérisé en ce qu'**on utilise en tant que composé d'un métal alcalin ou d'un métal alcalino-terreux le butyllithium, NaHCO₃ ou Na₂CO₃.

5. Procédé selon l'une des revendications précédentes, **caractérisé en ce qu'**on utilise par groupe NH de la triazine 0,05 à 1,2 équivalents en moles de composés d'un métal alcalin et d'un métal alcalino-terreux, de préférence 0,5 à 1,2 équivalents en moles, d'une manière particulièrement préférée 0,8 à 1,2 équivalents en moles.

6. Procédé selon l'une des revendications précédentes, **caractérisé en ce que** la réaction est mise en oeuvre à des températures de 0 à 200°C.

7. Procédé selon l'une des revendications précédentes, **caractérisé en ce que** pendant la réaction la température suit un profil de température.

8. Procédé selon la revendication 7, **caractérisé en ce que** la réaction est lancée à de basses températures, en particulier à des températures comprises entre 0 et 20°C, puis on élève la température jusqu'à une température finale sélectionnée, en particulier des températures comprises entre 20 et 200°C.

9. Procédé selon l'une des revendications précédentes, **caractérisé en ce qu'**on utilise par équivalents en moles de groupes NH de la triazine 0,7 à 10,0 moles, de préférence 0,9 à 7,0, de chloroformiate.

10. Procédé selon l'une des revendications précédentes, **caractérisé en ce que** la réaction est mise en oeuvre dans un solvant ou dans des conditions où le chloroformiate selon la formule (V) et/ou la formule (VI) joue le rôle de solvant.
